(19) Europäisches Patentamt
European Patent Office
Office européen des brevets

(11) Publication number: **0 340 778**
**A2**

(12) **EUROPEAN PATENT APPLICATION**

(21) Application number: 89108099.6

(22) Date of filing: 05.05.89

(51) Int. Cl.4: C07D 473/00 , C07D 405/04 , C07D 405/14 , A61K 31/505 , A61K 31/52

(30) Priority: 06.05.88 US 190809
07.04.89 US 333449

(43) Date of publication of application:
08.11.89 Bulletin 89/45

(84) Designated Contracting States:
AT BE CH DE ES FR GB GR IT LI LU NL SE

(71) Applicant: Bristol-Myers Company
345 Park Avenue
New York New York 10154(US)

(72) Inventor: Starrett, John E., Jr.
23 Hawks Nest Circle
Middletown Connecticut 06457(US)
Inventor: Mansuri, Muzammil M.
1320 Deer Run Road
Cheshire Connecticut 06410(US)
Inventor: Martin, John C.
40 Brookside Place
Cheshire Connecticut 06416(US)

(74) Representative: Kinzebach, Werner, Dr. et al
Patentanwälte Reitstötter, Kinzebach und Partner Sternwartstrasse 4 Postfach 86 06 49
D-8000 München 86(DE)

(54) **Prodrugs of 2',3'- Didehydro-2',3'-Dideoxynucleosides.**

(57) There are disclosed novel prodrugs of 2',3'-didehydro-2',3'-dideoxynucleosides, for example, of 2',3'-didehydro-2',3'-dideoxythymidine. The prodrugs are hydrolyzable under physiological conditions to yield compound which are useful as antiviral agents, especially as agents effective against the human immunodeficiency viruses (HIV).

EP 0 340 778 A2

# PRODRUGS OF 2′,3′-DIDEOXY-2′,3′-DIDEHYDRONUCLEOSIDES

## BACKGROUND OF THE INVENTION

### FIELD OF THE INVENTION

This invention relates to novel prodrugs of 2′,3′-didehydro-2′,3′-dideoxynucleosides which are useful as antiviral agents, especially as agents effective against the human immunodeficiency viruses (HIV). This invention also relates to a pharmaceutical composition containing the above prodrugs and to the anti-HIV use of said compositions.

### DESCRIPTION OF THE BACKGROUND AND RELATES REFERENCES

Acquired immunodeficiency syndrome (AIDS) is the result of an infection by human immunodeficiency virus(es) (HIV).[1] This retrovirus shows a specific tropism for the helper/inducer T cells[2] leading to their depletion. The resultant immunosuppression predisposes HIV patients to life-threatening opportunistic infections.

Although at present there is no cure for AIDS, one nucleoside derivative, 3′-azido-3′-deoxythymidine (AXT, Retrovir™), has already proved to be an efficacious agent in the treatment of AIDS in clinical trials and has been licensed by the appropriate regulatory agency for use in patients with AIDS[3]. A number of other chemical and biological agents have been reported to have biological activity against HIV. 2′,3′-Dideoxycytidine (ddC), 2′,3′-dideoxyadenosine (ddA),[4] 2′,3′-didehydro-2′,3′-dideoxycytidine (d4C),[5] suramine and its analogs,[6] ribavarin,[7] foscarnet,[8] HPA-23,[9] d-penicillamine,[10] castanospermine,[11] fusidic acid,[12] 3′-azido-3′-deoxyguanosine (AZG),[13] 3′-fluoro-3′-deoxythymidine (Fddt)[14] are all reported to be active against HIV.

A number of reports have appeared in the literature which have shown that 2′,3′-didehydro-2′,3′-dideoxythymidine (d4T) possesses in vitro activity against HIV in several cell lines.[15]

J.P.H. Verheyden and J.C. Martin, U.S. Patents No. 4,612,314 and 4,609,661, disclose substituted 9-(1- or 3-monoacyloxy- or 1,3-diacyloxy-2-propoxymethyl)purines as prodrugs of the antiviral compound, DHPG.

J.C. Martin et al., J. Pharm. Sci. 76 (2), 180-184 (1987), disclose mono-O-, di-O-, and $N^2$acyl derivatives of 9-[(1,3-dihydro-2-propoxy)methyl]guanine (DHPG).

V. Skaric and J. Matuliz-Adamic, Helv. Chim. Acta, 63, 2179 (1980) disclose 5′-O-acetyl-2′,3′-didehydro-2′,3′-dideoxythymidine.

S. David and G. deSennyey, Carbohydrate Research, 82, 45-49 (1980), disclose 5′-O-benzoyl-2′,3′-didehydro-2′,3′-dideoxyuridine.

R. Mengel and J. M. Seifert, Tetrahedron Lett., No. 48, 4203-4206 (1977), disclose a dipivaloyladenosine compound.

J. Lim et al., Antimicrobial Agents and Chemotherapy, July 1987, 998-1001 disclose methoxyacetate and ethoxypropionate esters of cyclaradine as prodrugs.

P.F. Torrence et al., Abstracts of the 2nd International Conference on Antiviral Research, Williamsburg, VA., 10-14 April 1988, page 118, disclose heteroarylcarbonyloxy esters, namely the 1,4-dihydro-1-methyl-3-pyridinylcarbonyloxy ester, as a prodrug of AZT.

T.J. Bardos et al., U.S. Patent 4,468,384 disclose the use of prodrugs of 4-deoxopyrimidines as antiviral agents.

## SUMMARY OF THE INVENTION

This invention is a novel 5′-O-acyl-2′,3′-didehydro-2′,3′-dideoxynucleosides of the formula

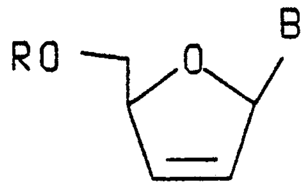

( I )

wherein B and R are a base and an acyl group, respectively, as defined below and a pharmaceutical composition containing the same and the antivral use of the same.

DETAILED DESCRIPTION OF THE INVENTION

In one generic aspect, this invention is a compound having the formula

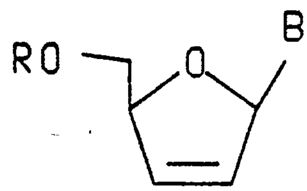

( I )

wherein B is a substituent derived from a member selected from the group of unsubstituted and substituted bases consisting of purine, aza-purine, deaza-purine, pyrimidine, aza-pyrimidine, deaza-pyrimidine, 2-hydroxy, thiol, or amino)-4-thio pyrimidine, 2-(hydroxy, thiol, or amino) pyrimidine, and triazole ring bases; and R is selected from H and a member selected from the group of physiologically hydrolyzable chemical groups consisting of alkylcarbonyl, arylcarbonyl, heteroarylcarbonyl, alkoxycarbonyl, aryloxycarbonyl, and heteroaryloxycarbonyl groups wherein the alkyl moiety consists of unsubstituted and substituted, straight-chain and branched-chain and cyclic alkyl groups having 1-20 carbon atoms, unsubstituted and substituted, straight chain and branched chain and cyclic alkenyl groups having 1-20 carbon atoms and phenalkyl groups wherein the alkyl moiety contains 1-3 carbon atoms and the phenyl moiety is unsubstituted or substituted and the aryl moiety consists of unsubstituted and substituted phenyl groups; provided that when B is thymidyl then R is not acetyl and when B is uridyl then R is not benzoyl and when R is H then B is selected from 2-hydroxy-4-thiopyrimidin-1-yl and 4-deoxopyrimidine.

In another aspect, this invention is a pharmaceutical composition comprising an antiviral effective amount of the compound having Formula I according to this invention and a pharmaceutically acceptable carrier.

In yet another aspect, this invention is a method for reducing the infectivity of the human immunodeficiency viruses by contacting said viruses with an antiviral effective amount of the compound having Formula I according to this invention.

As is described above, in one aspect this invention concerns prodrug esters which are 5′-O-acyl-2′,3′-didehydro-2′,3′-dideoxynucleosides wherein the base component B is derived from a member selected from the group of bases consisting of unsubstituted and substituted purine, aza-purine, deaza-purine, pyrimidine, aza-pyrimidine, deaza-pyrimidine, and triazole ring bases. Preferably, the base is selected from purine and pyrimidine bases. More preferably, the base is a pyrimidine base. Most preferably, the base is a pyrimidine base selected from thymine, cytosine, and uracil.

In another preferred embodiment, this invention concerns prodrugs which are 2′,3′-didehydro-2′,3′-dideoxynucleoside derivatives wherein, according to Formula I, R is H and B is selected from 2-hydroxy-4-thiopyrimidines (Formula III below, $R^3 = OH$, $R^5 = SH$), 2-hydroxypyrimidines (Formula III below, $R^3 = OH$, $R^5 = H$). The remaining ring atoms may be unsubstituted or substituted as described for pyrimidine bases having Formula III below.
These bases are produced as described in Torrence et al. and Bardos et al., respectively, mentioned above.

Suitable unsubstituted and substituted purine bases from which the component B may be derived include those purine bases represented Formula II

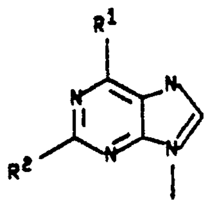

(II)

wherein $R^1$ and $R^2$ may be the same or different and are selected from hydrogen, hydroxy, halo (F, Cl, Br), amino, monoalkylamino, dialkylamino, alkoxy and cyano groups wherein the alkyl moiety is selected from $C_1$-$C_3$ alkyl groups.

Suitable unsubstituted and substituted 1-pyrimidyl bases comprising component B include those represented by Formula III

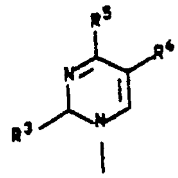

(III)

wherein $R^3$ is selected from hydroxy, amino and sulfhydryl groups; $R^5$ is selected from hydrogen, hydroxy and sulfhydryl, and amino group; and $R^6$ is selected from hydrogen, $C_1$-$C_1$ alkyl, $C_2$-$C_3$ alkenyl, $C_2$-$C_3$ haloalkenyl having from 1 to 5 halo groups as defined herein, $C_2$-$C_3$ alkynyl, alkoxy wherein the alkyl moiety has 1-3 carbon atoms, cyano and halo (F, Cl, Br and I).

When derived from purine bases, representative of B are the following:

6-aminopurin-9-yl (aden-9-yl)
2-aminopurin-9-yl
2,6-diaminopurin-9-yl
2-amino-6-hydroxypurin-9-yl (guanin-9-yl)
6-hydroxypurin-9-yl (hypoxanthine)

In addition to the above, the B component may be 2-halopurin-9-yl, 6-halopurin-9-yl, or 2,6-dihalopurin-9-yl.

When derived from pyrimidine bases, representative of B are the following:

2,4-dihydroxyprimidin-1-yl (uridyl)
5-methyl-2,4-dihydroxypyrimidin-1-yl (thymidyl)
5-ethyl-2,4-aminopyrimidin-1-yl
2-hydroxy-4-aminopyrimidin-1-yl (cytidyl)
5-vinyl-2,4-dihydroxypyrimidin-1-yl
5-halovinyl-2,4-dihydroxypyrimidin-1-yl
5-halomethyl-2,4-dihydroxypyrimidin-1-yl
5-haloethyl-2,4-dihydroxypyrimidin-1-yl

The above-mentioned 5-methyl and 5-ethyl substituents are representative of 5-alkyl substituents and the 5-vinyl substituent is representative of 5-alkenyl substituents. Examples of halo-groups ofthe 5-halovinyl (or 5-haloalkenyl) group include 1 to 4 F, Cl, and Br groups.

As is described above, in the case when R is other than H, then R is a member selected from the group of physiologically hydrolyzable ester groups consisting of alkylcarbonyl, arylcarbonyl, alkoxycarbonyl and aryloxycarbonyl groups wherein the alkyl moiety consists of unsubstituted and substituted, straight-chain and branched-chain and cyclic alkyl groups having 1-20 carbon atoms, unsubstituted and substituted, straight-chain and branched-chain and cyclic alkenyl groups having 1-20 carbon atoms, unsubstituted and substituted phenyl, and phenalkyl groups wherein the alkyl moiety contains 1-3 carbon atoms and the phenyl moiety is unsubstituted or substituted, provided that when B is thymidyl then R is not acetyl and when B is uridyl then R is not benzoyl.

By the expression "$C_1$-$C_{20}$ alkyl" and "$C_1$-$C_{20}$ alkoxy" wherein the alkyl moiety is $C_1$-$C_{20}$, more

particularly unsubstituted $C_1$-$C_{20}$ alkyl, is meant straight-chain or branched-chain or cyclic alkyl (monovalent hydrocarbon) groups having a total of 1-20 carbon atoms. Examples of suitable straight-chain alkyl groups include methyl, ethyl, propyl, butyl, pentyl, hexyl, heptyl, n-octyl, dodecyl, palmityl and the like groups. Examples of suitable branched-chain alkyl groups include isopropyl, sec-butyl, t-butyl, 2-methylbutyl, 2-pentyl, 3-pentyl and the like groups. Examples of suitable cyclic alkyl groups include cyclopropyl, cyclobutyl, cyclopentyl and cyclohexyl groups. The alkyl groups may be substituted, generally with 1 or 2 substituents, wherein the substituents are independently selected from halo, hydroxy, alkoxy, amino, mono- and dialkylamino, nitro, carboxyl, alkoxycarbonyl, and cyano groups. By the expression "phenalkyl" is meant benzyl, phenethyl (phenylethyl) and phenylpropyl groups wherein the phenyl moiety may be substituted. When substituted, the phenyl group or the phenyl moiety of the phenalkyl group may contain, independently, from 1 to 3 alkyl, hydroxy, alkoxy, halo, amino, mono- and dialkylamino, nitro, carboxyl, alkoxycarbonyl, and cyano groups.

By the expression "alkenyl" is meant unsubstituted or substituted, straight-chain or branched-chain or cyclic monovalent hydrocarbon substituents having 2 to 20 carbon atoms and having one double bond. Examples of suitable "alkenyl" groups include vinyl (ethenyl), 1-propenyl, alkyl, i-butenyl, pentenyl, hexenyl, n-decenyl and c-pentenyl and the like.

By the expression "heteroaryl" is meant aryl groups containing a heteroatom such as, for example in a preferred embodiment, 1,4-dihydro-1-methyl-3-pyridimyl which is mentioned above. See Torrence et al., supra.

As used herein, the expression "halo" is meant in the conventional sense to include F, Cl, Br, and I.

In more preferred embodiments, R is selected from the group consisting of

| | |
|---|---|
| $CH_3C(O)$ | (acetyl) |
| $C_6H_5C(O)$ | (benzoyl) |
| $(CH_3)_3CC(O)$ | (pivaloyl) |
| $CH_3OCH_2C(O)$ | (methoxyacetyl) |
| $C_7H_{15}C(O)$ | (heptoyl) |
| $C_{15}H_{31}C(O)$ | (palmitoyl) |
| $CH_3OC(O)$ | (methoxycarbonyl) |
| $C_8H_{17}OC(O)$ | (n-octyloxycarbonyl) |
| c-$C_6H_{11}OC(O)$ | (cyclohexyloxycarbonyl) |
| $(CH_3)_3COC(O)$ | (t-butoxycarbonyl) . |
| 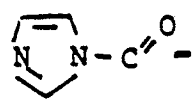 | (imidazolylcarbonyl) |
| 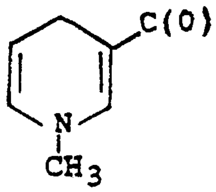 | (1,4-didhydro-1-methyl-3-pyridinylcarbonyl) |

Stated another way and to summarize, the present invention provides compounds of Formula I wherein a compound having Formula I wherein R is selected from H and a physiologically hydrolyzable ester group consisting of alkanoyl, cycloalkanoyl, cycloalkylalkanoyl, alkenoyl, cycloalkylalkenoyl, aroyl, aralkanoyl, aralkenoyl, heteroaroyl, alkoxycarbonyl, aryloxycarbonyl, or heteroaryloxycarbonyl groups wherein the alkanoyl, cycloalkanoyl, cycloalkylalkanoyl, alkenoyl, and cycloalkylalkenoyl each have up to 20 carbon atoms and 1 or 2 optional substituents selected from halo, hydroxy, alkoxy, amino, alkylamino, dialkylamino, nitro, carboxyl, alkoxycarbonyl, and cyano, and the aroyl, aralkanoyl, and aralkenoyl, each have up to 20 carbon atoms including from 6 to 10 carbon atoms in the aryl portion each aryl portion having optional substituents selected from 1 to 3 alkyl groups, hydyroxy, alkoxy, halo, amino, alkylamino, dialkylamino,

nitro, carboxyl, alkoxycarbonyl and cyano;

B is a nitrogen heterocycle attached through a ring nitrogen atom thereof and selected from 9-purinyl of Formula II and 1-pyrimidinyl of Formula III.

In Formulas II and III above, $R^1$ and $R^2$ are independently selected from hydrogen, hydroxy, fluorine, chlorine, bromine, amino, alkylamino, dialkylamino, alkoxy, and cyano and each of said alkyl and alkoxy groups has from 1 to 3 carbon atoms,

$R^3$ is hydroxy, amino, or sulfhydryl,

$R^5$ is hydrogen, hydroxy, sulfhydryl, or amino, and

$R^6$ is hydrogen, $C_{1-3}$ alkyl, $C_{2-3}$alkenyl, $C_{2-3}$ haloalkenyl having 1 to 5 halogen atoms, $C_{2-3}$ alkynyl, $C_{1-3}$ alkoxy, cyano, fluorine, chlorine, bromine, or iodine provided that when B is thymine R is other than acetyl, and when B is uridine R is other than benzoyl, and when R is H, then B is a pyrimidine of Formula III wherein $R^5$ is hydrogen or sulfhydryl.

As is mentioned above, the compound according to this invention may be provided as a pharmaceutical composition containing an antiviral effective amount, more particularly an anti-HIV effective amount, of the compound according to this invention having Formula I and a pharmaceutically acceptable carrier.

The pharmaceutical carrier may be solid or liquid to provide solid or liquid compositions. Solid form compositions suitable for oral administration include powders, tablets, capsules, caplets, dispersible granules, and cachets. Suitable solid carriers include at least one carrier substance which may function only as a carrier or may in addition serve a further function such as a diluent, flavoring agent, solubilizer, lubricant, suspending agent, binder, tablet disintegrating agent, encapsulating agent and the like. Inert solid carriers include, to name but a few, magnesium carbonate and stearate, talc, sugar, lactose, pectin, dextrin, starch, gelatin, cellulosic materials, and the like. The compounds according to the invention may be provided as sterile soluble compounds or compositions, including solutions and suspensions and emulsions thereof, which can be dissolved in sterile water or other liquid medium for oral administration or for parenteral administration. Examples of liquid carriers suitable for oral administration include water, alcohol, polypropylene glycol, polyethylene glycol and mixtures of two or more of the above. Examples of liquid carriers suitable for parenteral use include water-for-injection, physiological saline, and other suitable sterile injection media. Suitable buffers for use with the liquid carrier to provide, generally, a suitable buffered isotonic solution include trisodium orthophosphate, sodium bicarbonate, sodium citrate, N-methylglucamine, L(+)-lysine, and L(+)-arginine to name but a few representative buffering agents.

For aerosol administration, the carrier may be a combination of a suitable surfactant and propellant as the vehicle for the finely divided solid form or solution form of the active ingredient having Formula I.

An art-recognized compendium of pharmaceutical compositions including methods of preparation and ingredients is Remington's Pharmaceutical Sciences by E. W. Martin (Mark Pubb. Co., 15 Ed., 1975).

The pharmaceutical composition will contain an amount of active component, that is, compound of Formula I or mixture thereof with other anti-viral or anti-HIV active ingredient, which may be varied or adjusted, widely depending upon the particular application, the form, the potency of the particular compound used, and the desired concentration of compound in the composition. Generally, the amount of active component in the pharmaceutical composition will range between about 0.5-90% by weight based on total weight of composition.

As mentioned above, this invention is also a method for eliminating or reducing the infectivity ofthe human immunodeficiency viruses by contacting therewith an anti-HIV effective amount ofthe compound of Formula I, generally provided as a pharmaceutical composition described above.

In therapeutic use for treating a mammalian host, for example a human patient or an experimental animal host, affected by the human immunodeficiency viruses (HIV), the compounds of this invention will be administered in an amount effective to eliminate or reduce the infectivity of HIV that is, an anti-HIV effective amount of dosage. Generally, the anti-HIV effective amount will be in the range of about 1.0 to about 200 mg/kg of total body weight/day, more preferably about 1.0 to 30.0 mg/kg total body weight/day, most preferably about 5.0 to 20.0 mg/kg total body weight/day. It is to be understood that the actual preferred dosage of compound will vary widely depending upon the requirements ofthe animal being treated, the particular animal host and situs and disease being treated, the composition being used, and the route of administration. Many factors will be taken into account as is known by one skilled in the art to which this inventio pertains including, for example, age, body weight and sex of the animal host; diet; time of administration; rate of excretion; condition ofthe host; severity ofthe disease; and the like. Administration may be carried out simultaneously or periodically within the maximum tolerated dose. Optimal administration (or application) rates for a given set of conditions may be readily ascertained by those skilled in the art using conventional dosage determination tests.

The compounds of Formula I may be prepared from the corresponding 5′-hydroxy-2′,3′-didehydro-2′,3′-dideoxynucleosides (Formula I wherein R = H) by reacting the 5′-hydroxy group with the desired acyl anhydride or acyl chloride according to methods well known for protecting hydroxy groups. Several suitable methods are described above in the section "Description of the Background and Related References."

The following examples illustrate but a few representative embodiments ofthe compound having Formula I according to this invention and the preparation ofthe same and are set forth to teach those skilled in the pertinent art how to practice this invention and are not to be construed as limiting in scope. All parts and percentages are by weight and temperatures are in degrees Celsius unless otherwise specified. The structural formulas are set forth in TABLE 1.

The physiologically hydrolyzable esters according to this invention function as "pro-drugs" by being hydrolyzed in the body of the mammalian host, e.g., an experimental animal and a human patient, to yield the corresponding 2′,3′-didehydro-2′,3′-dideoxynucleoside per se. Early data reported in the pertinent literature tends to show that the 2′,3′-didehydro-2′,3′-dideoxynucleosides derived from purine bases are less active or potent than those derived from pyrimidine bases. See DeClercq et al., J. Med. Chem., 30, 1270 (1987). Thus, the pro-drugs ofthe latter are more preferred than those of the former. In addition to providing the corresponding 2′,3′-didehydro-2′,3′-dideoxynucleoside under physiological hydrolysis conditions, the compound according to this invention having Formula I may afford other advantages such as advantages in formulation and prolonged and sustained action of active ingredient, stability in formulation and upon storage, and the like.

The most preferred embodiments ofthe present invention are the following esters of 2′,3′-didehydro-2′,3′-dideoxy thymidine:
acetyl,
benzoyl,
methoxyacetyl,
pivaloyl,
methoxycarbonyl,
imidazolylcarbonyl,
cyclohexyloxycarbonyl, and
n-octyloxycarbonyl.

## DESCRIPTION OF SPECIFIC EMBODIMENTS

Abbreviations:

| | |
|---|---|
| d4T | 2′,3′-didehydro-2′,3′-dideoxythymidine |
| imidazoyl d4T | 5′-(1-imidazolylcarbonyl)-2′,3′-didehydro-2′,3′-dideoxythymidine |

**Example 1:**

**5′-O-Acetyl-2′,3′-didehydro-2′,3′-dideoxythymidine (v.Skaric et al., loc. cit.)**

To a stirred solution of 10.0 g (44.6 mmol) of d4T in 100 mL of pyridine was added 4.10 mL (58.0 mmol) of acetyl chloride. The mixture was stirred at 50°C for 1 h, cooled and diluted with 1 L of toluene. The solvents were removed in vacuo. The yield of product, recrystallized from ethanol was 9.3 g. $^1$H NMR (360 MHz) 9.01 (1H, s, NH), 7.23 (1H, s, C6H), 6.99 (1H, d, J=1.1 Hz, C1′H), 6.24 (1H, d, J=5.8 Hz, C2′H), 5.88 (1H, d, J=5.5 Hz, C3′H), 5.02 (1H, s, C4′H), 4.3 (2H, m, C5′H$_2$), 2.07 (3H, s, CH$_3$ acetate), 1.90 (3H, s, C5CH$_3$). MS m/z (relative intensity) 533 (M$^+$, 3), 347 (40), 287 (50), 155 (40), 127 (100), 81 (80). IR (KBr) cm$^{-1}$ 3010, 1740, 1700, 1470, 1260, 1230. Anal. calculated $C_{12}H_{14}N_2O_5$ C 54.12 H 5.30 N 10.52; found C 53.97 H 5.36 N 10.45.

**Example 2:**

**5′-O-Benzoyl-2′,3′-didehydro-2′,3′-dideoxythymidine.**

To a solution of 10.0 g (44.6 mmol) of d4T in 290 mL of pyridine was added 8.2 mL (71.4 mmol) of benzoyl chloride. The mixture was heated at 50° C for 6 h, cooled, and half of the volatiles were removed in vacuo. The residue was poured in 800 mL of ice water and the resulting precipitate was collected, washed with ice water and air dried. The solid was recrystallized from ethanol to give 12.87 g of title product. $^1H$ NMR (300 MHz, DMSO) 11.35 (1H, s, NH), 7.91 (2H, d, J=7.8 Hz, Ph), 7.65 (1H, t, J=7.2 Hz, Ph), 7.51 (2H, d, J=7.8 Hz, Ph), 7.09 (1H, s, C6H0, 6.78 (1H, s, C1′H), 6.50 (1H, d, J=6.1 Hz, C2′H), 6.02 (1H, d, J=6.1 Hz, C3′H), 5.08 (1H, s, C4′H), 4.48 (2H, m, C5′$CH_2$), 1.33 (3H, s, C5$CH_3$). MS m/z relative intensity) 329 (M+H, 12), 155 (45), 127 (100), 81 (85). IR (KBr) $cm^{-1}$ 3100-3000, 1710, 1460, 1280. Anal. calculated $C_{17}H_{16}N_2O_5$ C 62.20 H 4.92 N 8.54; found 62.25 H 4.91 N 8.28.

**Example 3:**

**5′-O-Methoxyacetyl-2′,3′-didehydro-2′,3′-dideoxythymidine.**

To a solution 4.50 g (20.1 mmol) of d4T in 50 mL of pyridine was added 2.45 mL (26.8 mmol) of methoxyacetyl chloride. The reaction was stirred at 22° C for 27 h, diluted with 200 mL of toluene and stripped to dryness in vacuo. The residue was recrystallized from ethanol. NMR analysis of the product indicated presence of pyridine hydrochloride. The solid was suspended in 100 mL of water, collected by filtration, and washed with absolute ethanol to give 2.50 g of methoxyacetate. $^1$NMR (300 MHz, $CDCl_3$) 9.27 (1H, s, NH), 7.14 (1H, d, J=1.3 Hz, C6H), 6.96 (1H, s, C1′H), 6.23 (1H, d, J=6.0 Hz, C2′H), 5.88 (1H, d, J=6.1 Hz, C3′H), 5.01 (1H, s, C4′H), 4.3 (2H, m, C5′H), 4.02 (2H, m, $OCH_2$), 3.39 (3H, s, $OCH_3$), 1.88 (3H, s, C5$CH_3$). MS m/z (relative intensity) 297 (M+H), 40), 251 (20), 207 (40), 155 (50), 127 (100), 81 (100). IR (KBr) $cm^{-1}$ 3200-3000, 1760, 1700, 1480, 1260. Anal. calculated $C_{13}H_{16}N_2O_6$ C 52.70 H 5.44 N 9. 46; found C 52.59 H 5.40 N 9.11.

**Example 4:**

**5′-O-Pivaloyl-2′,3′-didehydro-2′,3′-dideoxythymidine**

To a solution of 1.84 g (8.21 mmol) of d4T in 20 mL of pyridine was added 1.31 mL (10.67 mmol) of pivaloyl chloride. The mixture was heated at 65° C for 3h, cooled and diluted with 100 mL of toluene. The solvents were removed in vacuo and the residue was recrystallized from isopropyl alcohol to give 0.72 g of pivaloate. $^1H$ NMR (300 MHz, DMSO) 11.40 (1H, s, NH), 7.19 (1H, s, C6H), 6.75 (1H, s, C1′H), 6.37 (1H, d, J=6.0 Hz, C2′H), 6.00 (1H, d, J=6.0 Hz C3′H), 4.91 (1H, s, C4′H), 4.16 (2H, m, C5′$H_2$), 1.74 (3H, s, C5$CH_3$), 1.10 (9.H, s, t-butyl). MS m/z (relative intensity) 309 (M+H, 30), 207 (70), 155 (60), 127 (100), 81 (80). IR (KBr) $cm^{-1}$ 3100-3000, 1735, 1700, 1470, 1135. Anal. calculated $C_{15}H_{20}N_2O_5.04H_2O$ C 58.31 H 6.63 N 9.07; found C 57.91 H 6.72 N 9.08.

**Example 5:**

**5′-O-Methoxycarbonyl-2′,3′-didehydro-2′,3′-dideoxythymidine.**

A suspension of 1.21 g (3.80 mmol) of imidazoyl d4T in 30 mL of methanol was heated to reflux for 15 minutes. Upon initially reaching reflux temperature, the reaction became homogeneous. The flask was cooled to room temperature (22° C), then placed in an ice bath at 0° C for 30 minutes. The resulting precipitate was collected and washed with cold methanol. The filtrate was cooled at 0° C for 16 h and the resulting solid was collected and washed with cold methanol. The solids were combined to give 0.85 g of methoxycarbonyl d4T. $^1H$ NMR (300 MHz, $CDCl_3$) 8.55 (1H, s, NH), 7.35 (1H, s, C6H), 7.03 (1H, m, C1′H), 6.27 (1H, m, C2′H), 5.85 (1H, d, J=6.0 Hz, C3′H), 4.99 (1H, m, C4′H), 4.36 (2H, m, C5′$H_2$), 3.77 (3H, s, $OCH_3$), 1.87 (3H, s, C5$CH_3$). MS m/z (relative intensity) 283 (M+H, 20), 207 (40), 155 (50), 127 (100), 81

(100). IR (KBr) $cm^{-1}$ 2975, 1750, 1700, 1280, 1260. Anal. calculated $C_{12}H_{14}N_2O_6$ C 51.06 H 5.00 N 9.93; found C 50.90 H 4.89 N 9.87.

**Example 6**

**5′-O-Cyclohexyloxycarbonyl-2′,3′-didehydro-2′,3′-dideoxy-thymidine**

A suspension of 1.00 g (3.14 mmol) of imidazoyl d4T in 20 mL of cyclohexanol was heated at 100°C for 18 h, after which time the reaction was homogeneous. The reaction was cooled and the solvents were removed in vacuo at 50°C (0.1 mm). The residue was purified on a 50 mm flash chromatography column, eluting with 2% MeOH-$CH_2Cl_2$ (1000 mL), 5% MeOH- $CH_2Cl_2$, (500 mL), then 10% MeOH-$CH_2Cl_2$ (500 mL). The fractions containing the desired product were combined and the solvent was removed to give an oil which still contained cyclohexanol by NMR. The oil was suspended in 30 mL of water and the solvents were removed by freeze-drying (0.005 mm) for 16 h to give 0.51 g of cyclohexyloxycarbonate. $^1H$ NMR (200 MHz, $CDCl_3$) 8.20 (1H, s, NH), 7.45 (1H, s, C6H), 7.08 (1H, m, C1′H), 6.30 (1H, m, C2′H), 5.85 (1H, m, C3′H), 4.05 (1H, m, C4′H), 4.6 (1H, m, CHO), 4.4 (2H, m, C5′$CH_2$), 2.1-1.0 (13H, m, C5$CH_3$ + cyclohexyl $CH_2$). MS m/z (relative intensity) 351 (M+H, 55), 207 (50), 155 (40), 127 (50), 81 (100). IR (KBr) $cm^{-1}$ 2975, 1750, 1700, 1280, 160. Anal. calculated $C_{17}H_{22}N_2O_6$ C 58.27 H 6.33 N 8.00; found C 58.23 H 6.05 N 7.90.

**Example 7:**

**5′-O-(1-Imidazoylcarbonyl)-2′,3′-didehydro-2′,3′-dideoxythymidine**

To a solution of 4.50 g (0.0201 mol) of d4T in 50 mL of DMF was added 5.67 g (0.035 mol) of carbonyldiimidazole. The reaction was stirred at 22°C for 45 minutes, during which time a thick white precipitate formed. The solid was collected and washed with 5 mL of DMF. The filtrate was cooled to 0°C for 16 h, and the resulting solid collected and washed with cold DMF. The combined solid was dried in a vacuum dessicator for 16 h at 0.1 mm to give 5.50 g of imidazoate which contains about 5% imidazole (determined by NMR analysis). $^1H$ NMR (300 MHz, DMSO) 8.22 (1H, s, imid. H), 7.55 (1H, s, imid. H), 7.11 (1H, s, imid. H), 7.11 (1H, s, C6H), 6.77 (1H, d, J=1.9 Hz, C1′H), 6.49 (1H, d, J=6.0 Hz C2′H), d6.04 (1H, d, J=5.9 Hz, C3′H), 5.02 (1H, s, C4′H), 4.5 (2H, m, C5′$CH_2$), 1.53 (3H, s, C5$CH_3$). MS m/z (relative intensity) 319 (M+H, 100), 207 (10), 149 (20), 127 (20), 81 (25). IR (KBr) $cm^{-1}$ 3085, 1760, 1700, 1690, 1670, 1415, 1260.

**Example 8**

**5′-O-Benzoyl-2′,3′-dihydro-2′,3′-dideoxy-4-thio-thymidine**

A suspension of 5-O-benzoyl-2′,3′-didehydro-2′,3′-dideoxy-thymidine (12.6 g, 38.45 mmoles) and 2,4-bis(4-methoxy-phenyl)-1,3-dithia-2,4-diphosphetane-2,4-disulfide (Lawesson's reagent) 10.87 g, 26.9 mmoles) in chloroform (270 mL) was heated to reflux for 4 h. The homogeneous reaction mixture was concentrated and purified by flash column chromatography using methylene chloride/ethyl acetate (9:1) as eluent. The product was collected as a solid (10.56 g, 80%). Mp 171-173°C. $^1H$ NMR (300 MHz, $d_6$-DMSO) 12.76 (s, 1H, NH), 7.90 (d, 2H, ortho Ph), 7.64 (t, 1H, para Ph), 7.50 (t, 2H, meta Ph), 7.22 (s, 1H, H6), 6.74 (d, 1H, H1′), 6.52 (d, 1H, H3′), 6.04 (d, 1H, H2′), 5.12 (s, 1H, H4′), 4.45 (m, 2H, H5′) and 1.53 (s, 3H, $CH_3$). Anal. Calc. for $C_{17}H_{16}N_2O_4S$: C, 59.29; H,4.69; N 8.14. Found: C, 5915; H, 4.64; N. 8.10.

**Example 9**

**2′,3′-Didehydro-2′,3′,-dideoxy-4-thio-thymidine**

A solution of 5′-O-benzoyl-2′,3′-didehydro-2′3′-dideoxy-4-thio-thymidine (3.09 g, 9 mmole) in anhydrous methanol (145 mL) was warmed and sodium methoxide (0.54 g, 10 mmole) was added. The solution was heated to reflux for 30 min. The pH of the solution was monitered and kept at pH 8. The reaction mixture was allowed to cool and then concentrated. The product was purified by flash column chromatography, using methylene chloride/methanol (4.1) as eluent. The product was collected as a solid (2.1 g, 99%). Mp 127-129° C. $^1$H NMR (300 Mhz, $d_6$-DMSO) 12.7 (s, 1H, NH), 7.8 (s, 1H, H6), 6.76 (m, 1H, H1′), 6.38 (m, 1H, H3′), 5.90 (m, 1H, H2′), 5.03 (t, 1H, OH), 4.77 (s, 1H, H4′), 3.58 (m, 2H, H5′) and 1.88 (s, 3H, $CH_3$). Anal. Calc. for $C_{10}H_{12}N_2O_3S$: C, 47.83; h, 5.03; N, 11.16; Found: C, 48.16; H, 5.33; N, 11.08.

**Example 10**

**5′O-Benzoyl-2′,3′-didehydro-2′3′-dideoxyuridine**

2′,3′-Didehydro-2′3,′-dideoxyuridine (10 g, 47.6 mmol) in pyridine (250 mL) was treated with benzoyl chloride (68.1 mmol, 9.5 g, 7.9 ml) at 50-55° C, and the reaction stirred for 5 h. The pyridine was then removed by azeotropic distillation with toluene. The residue was dissolved in water and extracted out with ethyl acetate. This solution was then concentrated down to give the product (14.7 g, 95%). The compound was used in the following reation without further purification. $^1$H NMR (300 MHz, $d_6$-DMSO) 7.5-8.0 (m, 5H, aromatic), 7.35 (d, 1H, J=7.5 HZ, H-6), 6.8 (s, 1H, H-1′), 6.5 (d, 1H, H-3′), 6.05 d, 1H, H-2′), 5.15 (d, 1H, J - 7.5Hz, H-5), 5.1 (m, 1H, H-4′), and 4.5 (m, 2H, H-5′).

**Example 11**

**5′-O-Benzoyl-2′,3′-didehydro-2′,3′-dideoxy-4-thio-uridine**

5′-O-Benzoyl-2′,3′-didehydro-2′3′-didehydro-2′,3′-dideoxyuridine (15 g, 47.78 mmol) in chloroform (250 mL) was treated with Lawesson's reagent (23.89 mmol, 9.67g). The reaction mixture became homogenous after 10 minutes at reflux and was then heated at reflux for 6.5 h. The mixture was then concentrated using a stream of dry $N_2$. The residue was purified by flash column chromatography using methylene chloride/ethyl acetate (9:1) as eluent. The desired product was isolated as a bright yellow solid (9.8 g, 60%); mp 128-129° C; $^1$H NMR (300 MHz, $d_6$-DMSO) 7.90 (d, 2H, H1, H6 aromatic), 7.67 (T, 1H, H4 aromatic), 7.53 (t, 2H, H3, H5 aromatic), 7.27 (d, 1H, J=7.5 Hz, H6), 6.77 (s, 1H, H1′), 6.55 (d, 1H, H3′), 6.05 (d, 1H, H2′), 5.85 (d, 1H, J -7.5Hz, H5), 5.15 (s, 1H, H4′), 4.46 (m, 2H, H5′); $^{13}$C NMR (75 MHz, $d_6$-DMSO) 190.49 (C4), 165.55 (C=O), 148.03 (C2), 133.68, 129.40, 129.26, 128.95 (aromatic), 135.66 (C6), 134.08 (C3′), 126.50 (C2′),112.72 (C5), 90.20 (C1′), 84.56 (C4′), 65.26 (C5′); IR (KBr) cm-1 3350-3150, 3150-2800, 1725, 1615, 1470; MS m/z (relative intensity) 331 (M+H, 2.5), 283 (3.25), 129 (100), 81 (42). Anal. Calc. $C_{16}H_{14}N_2O_4S_1$ C 58.17 H 4.27 N 8.48 Found: C 57.44; H 4.03; N 8.38; K.F. = 2.18%

**Example 12**

**5′-O-Benzoyl-2′,3′-didehydro-2′,3′dideoxycytidine**

5′-O-Benzoyl-2′,3′-didehydro-2′,3′-dideoxy-4-thio-uridine (1 g, 2.9 mmole) was treated with methanolic ammonia (20 mL) in a metal bomb at 100° C for 1 h. The reaction mixture was concentrated and the residue purified by flash column. chromatography eluting with 10% MeOH/$CH_2Cl_2$. $^1$H NMR (300 MHz, $d_6$-DMSO) 7.93 (d, 2H, ortho Ph), 7.68 (t, 1H, para Ph), 7.54 (t, 2H, meta Ph), 7.34 (d, 1H, H6), 7.18 (d, 1H, NH), 6.89 (s, 1H, H1′), 6.44 (d, 1H, H3′), 6.00 (d, 1H, H2′), 5.42 (d, 1H, H5), 5.09 (br s, 1H, H4′), 4.47 (m, 2H, H5′); $^{13}$C NMR (75.5 MHz, $d_6$-DMSO) 165.66, 154.35, 140.79, 133.58, 132.73, 129.39, 129.18, 128.83, 12779, 94.34, 90.01, 83.73, 65.48

Anal. Calc for $C_{16}H_{15}N_3O_4.0.5H_2O$: C, 59.61; H, 4.69; N, 13.03.

Found: C, 59.68; H, 5.10; N, 12.49.

**Example 13**

**5′-3-Pyridinylcarbonyl)-2′3′-didehydro-2′3′-dideoxythymidine**

Under an atmosphere of nitrogen, to a stirred solution of 0.92 g (4.10 mmol) of d4T in 15 mL of dry pyridine was added 0.95 g (5.34 mmol) of nicotinyl chloride hydrochloride. The reaction was placed in an oil bath and heated at 70° C for 3h. The reaction was cooled and the solvents were removed under a gentle stream of nitrogen at 22° C for 16h. The crude residue was purified on a 50 mm flash column, eluting with 5%MeOH/$CH_2Cl_2$. The appropriate fractions were combined and evaporated to constant weight to give 1.07 g (79%) of 5′-(3-Pyridinylcarbonyl)-2′,3′-didehydro-2′,3′-dideoxythymidine. Mp. 179-181° C. $^1$H NMR ($CDCl_3$; 300 MHz) 9.20 (1H,d,J=2.2,H2″), 8.80 (1H,dd,J=6.9,1.7,H6″), 8.52 (1H,br s,NH), 8.25 (1H,m,H4″), 7.39 (1H,m,H5″), 7.02 (1H,s,H6), 6.97 (1H,m,H1′), 6.37 (1H,m,H3′), 5.93 (1H,m,H2′), 5.15 (1H,m,H4′), 4.57 (2H,d, J=3.7,H5′), 1.58 (3H,d,J=2,$CH_3$).

5′-(1-Methyl-3-pyridiniumcarbonyl)-2′,3′-didehydro-2′,3′-dideoxythymidine

To a suspension of 5′-(3-Pyridinylcarbonyl)-2′,3′-didehydro-2′,3′-dideoxythymidine (0.85 g, 2.6 mmol) in 30 mL of acetone was added 1.5 mL (24 mmol) of methyl iodide. The mixture was refluxed for 24h, cooled, and the solvents were removed under a gentle stream of nitrogen to afford 1.16 g of a bright yellow solid of 5′-(1-methyl-3-pyridinium-carbonyl)-2′,3′-didehydro-2′,3′-dideoxythymidine. $^1$H NMR (DMSO; 300 MHz) 9.51 (1H,s,H2″), 9.15 (1H,d,J=6.0,H6″), 8.92 (1H,d,J=8.1,H4″), 8.22 (1H,dd,J=7.8,6.2, H5″), 7.21 (1H,s,H6), 6.80 (1H,t,J=1.9,H1′), 6.51 (1H,d,J=6.1,H3′), 6.05 (1H,d,J=6.0,H2′), 5.10 (1H,br s,H4′), 4.55 (2H,m,H5′), 4.39 (3H,s,N1″$CH_3$), 3.28 (1H,s,exchangable, NH), 1.58 3H,s,C5$CH_3$).

5′-(1,4-Dihydro-1-methyl-3-pyridinylcarbonyl)-2′,3′-didehydro-2′,3′-dideoxythymidine

In a 250 mL round bottomed flask flushed with nitrogen and externally cooled in an ice bath was added solid 5′-(1-Methyl-3-pyridiniumcarbonyl)-2′,3′-didehydro-2′,3′-dideoxythymidine (1.03 g, 2.19 mmol), sodium hydrosulfite (1.90 g, 10.9 mmol), and sodium bicarbonate (0.92 g, 10.9 mmol). To this stirred mixture was added 100 mL of 4° C water which had been deaerated by bubbling nitrogen through the solution for 15 min. the ice bath was removed from the flask and the reaction was stirred for 20 min. The resulting solid was collected, washed with cold, deaerated water, and dried with suction for 5 min. The slightly yellow, wet solid was dried under vacuum for 12 h to give 0.53 g of 5′-(1,4-Dihydro-1-methyl-3-pyridinylcarbonyl)-2′,3′-didehydro-2′,3′-dideoxythymidine. Mp. 145-147° C. $^1$H NMR (DMSO$d_6$; 300MHz) 7.12 (1H,s,H6), 7.00 (1H,s,H1′), 6.75 (1H,m,H2″), 6.38 (1H,d,J=5.9,H3′), 5.94 (1H,d,J=6.0,H2′), 5.79 (1H,d,J=8.2,H6″), 4.92 (1H,br s,H4′), 4.69 (1H,dt,J=8.1,3.2,H5″), 4.18 (2H,m,H5′), 2.89 (5H,s, H4″ +N1″$CH_3$), 1.72 (3H,s,C5$CH_3$).

## Antiviral Assays

The anti-HIV/LAV activity of several compounds representative of this invention and of d4T for comparison was measured in cultures of CEM-F cells. The results are set forth in the table below. The CEM cells were infected with approximately 30 $TCID_{50}$ (50% tissue culture infectious dose) of HIV (LAV strain). The cells were then incubated for 45 min 37° C. The test compounds, in culture medium, were added at various concentrations to the infected cells and then incubated for a further 8 days. After 8 days the antiviral activity was evaluated in the culture media supernatant for p-24 gag protein by an enzyme capture assay (ELISA). The antiviral activity was expressed as the dose which inhibits 50% of the virus expression ($ID_{50}$ in μM) as detected by the assay described.

| Antiviral Activity | |
|---|---|
| Example | $ID_{50}$ ($\mu M$) |
| 1 | 3.2 |
| 3 | 0.4 |
| 4 | 4.8 |
| 5 | 3.2 |
| 6 | 0.6 |
| 8 | 0.5 |
| 11 | 0.5 |
| 12 | 0.1 |
| d4T | 0.2 |

# TABLE I

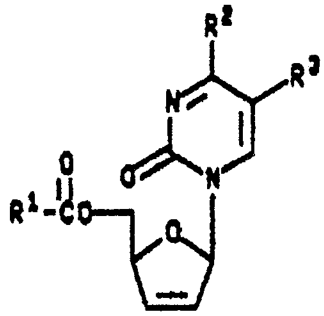

| EXAMPLE | $R^1$ | $R^2$ | $R^3$ |
|---|---|---|---|
| 1 | $CH_3$ | OH | $CH_3$ |
| 2 | $C_6H_5$ | OH | $CH_3$ |
| 3 | $CH_3O$ | OH | $CH_3$ |
| 4 | $(CH_3)_3C$ | OH | $CH_3$ |
| 5 | $CH_3OCH_2$ | OH | $CH_3$ |
| 6 | 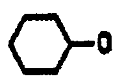 | OH | $CH_3$ |
| 7 | 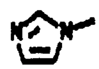 | OH | $CH_3$ |
| 8 | $C_6H_5$ | SH | $CH_3$ |
| 10 | $C_6H_5$ | OH | H |
| 11 | $C_6H_5$ | SH | H |
| 12 | $C_6H_5$ | $NH_2$ | H |
| 13 | 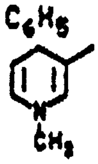 | OH | $CH_3$ |

9

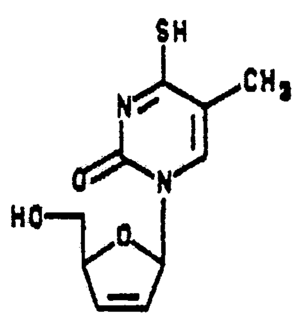

## References

1. (a) Barre-Sinoussi, F; Chermann, J.C.; Rey, R.; Nugeyre, M.T.; Chamaret, S.; Gruest, C.; Dauguet, C.; Axler-Blin, C.; Rouzioux, C.; Rozenbaum, W.; Montagnier, L. Science (Washington, D.C.) **1983,** 220, 868-871. (b) Broder, S.; Gallo, R.C. N. Engl. J. Med. **1984,** 311, 1292-1297. (c) Broder, S; Gallo, R.C. Annu. Rev. Immunol. **1985,** 3, 321-336.

2. Popovic, M.; Sarngadharan, M. G.; Read E.; Gallo, R.C. Science (Washington D.C.) **1984,** 224, 497-500. (b) Gallo, R. C.; Sarngadharan, M. G.; Popovic, M.; Shaw, G.M.; Hahn, B.; Wong-Stahl, F.; Robert-Guroff, M.; Salahaddian, Z., Markham, P.D. Prog. Allergy **1986,** 37, 1-45.

3. Fischl, M. A.; Richman, D.D.; Grieco, M. H.; Gottlieb, M. S.; Volberding, P. A.; Laskin, O. L.; Leedom, J. M.; Groopman, J. E.; Mildvan, D.; Schooley, R. T.; Jackson, G.G.; Durack, D. T.; King, D. New Engl. J. Med., **1987,** 317, 185.

4. Mitsuya, H.; Broder, S. Proc. Natl. Acad. Sci. U. S. A. **1986,** 83, 1911-1915.

5. (a) Lin, T.S.; Shinazi, R.; Chen, M. S.; Kinney-Thomas, E.; Prusoff, W. H. Biochem. Pharmacol. **1987,** 36, 311. (b) Balzarini, J.; Pauwels, R.; Herdewijn, P.; De Clercq, E.; Cooney, D.A.; Kang, G-J.; Dalal, M.; Johns, D.G.; Broder, S. Biochem. Biophys. Res. Comm. **1986,** 140, 735.

6. Cheson, B. D.; Levine, A. D.; Mildvan, D,; Kaplan, L. D.; Wolfe, P.; Rios, A.; Groopman, J.; Gill, P.; Volbdering, P. A.; Poiesz, B. J.; Gottlieb, M. S.; Holden, H.; Volsky, D. J.; Silver, S. S.; Hawkins, M. J. J. Amer. Med. Assoc. **1987,** 258, 1347.

7. (a) Balzarini, J.; Mitsuya, H.; De Clercq, E.; Broder, S. Int. J. Med., **1986,** 37, 451. (b) McCormick, J.B.; Getchell, J.B.; Mitchell, S. W.; Hicks, D. R. Lancet **1984,** ii, 1367.

8. (a) Sarin, P. S.; Taguchi, Y.; Sun, D.; Thornton, A.; Gallo, R.C.; Oberg, B. Biochem. Pharmacol. **1985,** 34, 4075. (b) Sandstrom, E. G.; Kaplan, J. C.; Byington, R.E.; Hirsch, M. s. Lancet **1985,** i, 480.

9. Lane, H.C.; Fauci, A. S. Ann. Intern. Med. **1985,** 103, 714.

10. Chandra, P.; Sarin, P.S. Arznrim-Forsch/Drug Res. **1986,** 36, 184.

11. Tyms, A.S.; Berrie, E.M.; Ryder, T. A.; Nash, R. J.; Hegarty, M. P.; Taylor, D. L.; Mobberley, M. A.; Davis, J. M.; Bell, E. A.; Jeffries, D. A.; Taylor-Robinson, D.; Fellows, L. E. Lancet **1987,** ii, 1025.

12. Faber, V.; Newell, A.; Dalgleish, A. G.; Malkovsky, M. Lancet **1987,** ii, 827.

13. (a) Hartmann, H.; Hunsmann, G.; Eckstein, F. Lancet **1987,** i, 40. (b) Baba, M.; Pauwels, R.; Balzarini, J.; Herdewijn, P.; De Clercq, E. Biochem. Biophys Res. Comm. **1987,** 145, 1080.

14. (a) Herdewijn, P.; Balzarini, J.; De Clercq, E.; Paules, R.; Baba, M.; Broder, S.; Vanderhaeghe, H. J. Med. Chem. **1987,** 30, 1270. (b) Mattes, E.; Lehmann, C.; Scholz, D.; von Janta-Lipinski M.; Gaertner, K.; Rosenthal, H. A.; Langen, P. Biochem. Biophs Res. Comm. **1987,** 148, 78. (c) Polski, B.; Gold, J. M. W.; Hardy, W.D.; Baron, P.A.; Zuckermann, E.E.; Chou, T-C.; Levine, S.M.; Flomenberg, N.; Wang, L.; Watanabe, K. A.; Fox, J. J.; Armstrong, D. 27th ICAAC **1987,** Abstract 368, p161.

15. (a) Lin, T. S.; Chen, M. S.; Gao, Y-S.; Ghazzouli, I.; Prusoff, W. H. J. Med. Chem. **1987,** 30, 440. (b) Lin, T.S.; Shinazi, R. F.; Prusoff, W. H. Biochem. Pharmacol. **1987,** 17, 2713. (c) Baba, M.; Pauwels, R.; De Clercq, E.; Desmyter, J.; Vandeputte, M. Biochem. Biophys. Res. Comm. **1987,** 142, 128. (d) Balzarini, J.; Kang, G-J.; Dalal, M.; Herdewjin, P.; De Clercq, E.; Broder, S.; Johns, D. G. Mol. Pharmacol. **1987,** 32, 162. (e) Hamamoto, Y.; Nakashima, H.; Matsui, T.; Matsuda, A.; Ueda, T.; Yamamoto, N. Amtimicrob. Agents Chemother. **1987,** 31, 907.

16. Horwitz, J.; Chua, J. in "Synthetic Procedures in Nucleic Acid Chemistry" (Vol. 1), Zorbach, W. W.; Tipson R. S. (eds); Interscience, New York, p. 344. Horwitz, J.; Chua, J.; Da Rooge, M. A.; Noel, M.; Klundt, I. L. J.Org. Chem. **1966,** 31, 205.

## Claims

1. A compound having Formula I

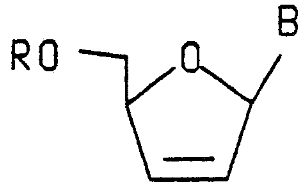

( I )

wherein R is selected from H and a physiologically hydrolyzable ester group consisting of alkanoyl, cycloalkanoyl, cycloalkylalkanoyl, alkenoyl, cycloalkylalkanoyl, aroyl, aralkanoyl, aralkenoyl, heteroaroyl, alkoxycarbonyl, aryloxycarbonyl, or heteroaryloxycarbonyl groups wherein the alkanoyl, cycloalkanoyl, cycloalkylalkanoyl, alkenoyl, and cycloalkylalkanoyl, each have up to 20 carbon atoms and 1 or 2 optional substituents selected from halo, hydroxy, alkoxy, amino, alkylamino, dialkylamino, nitro, carboxyl, alkoxycarbonyl, and cyano, and the aroyl, aralkanoyl, and aralkenoyl, each have up to 20 carbon atoms including from 6 to 10 carbon atoms in the aryl portion each aryl portion having optional substituents selected from 1 to 3 alkyl groups, hydroxy, alkoxy, halo, amino, alkylamino, dialkylamino, nitro, carboxyl, alkoxycarbonyl and cyano; B is a nitrogen heterocycle attached through a ring nitrogen atom thereof and selected from 9-purinyl of Formula II and 1-pyrimidinyl of Formula III

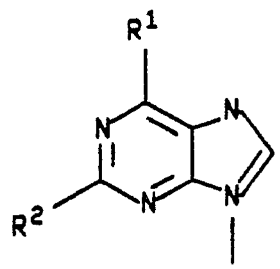

(II)

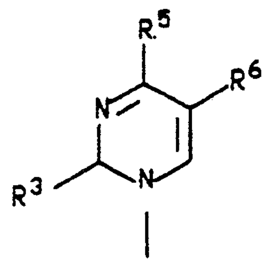

(III)

wherein

$R^1$ and $R^2$ are independently selected from hydrogen, hydroxy, fluorine, chlorine, bromine, amino, alkylamino, dialkylamino, alkoxy, and cyano and each of said alkyl and alkoxy groups has from 1 to 3 carbon atoms,
$R^3$ is hydroxy, amino, or sulfhydryl,
$R^5$ is hydrogen, hydroxy, sulfhydryl, or amino, and
$R^6$ is hydrogen, $C_{1-3}$ alkyl, $C_{2-3}$ alkenyl, $C_{2-3}$ haloalkenyl having 1 to 5 halogen atoms, $C_{2-3}$ alkynyl, $C_{1-3}$ alkoxy, cyano, fluorine, chlorine, bromine, or iodine provided that when B is thymine R is other than acetyl, and when B is uridine R is other than benzoyl, and when R is H, then B is a pyrimidine of Formula III wherein $R^5$ is hydrogen or sulfhydryl.

2. The compound of Claim 1 wherein R is hydrogen.
3. The compound of Claim 1 wherein R is other than hydrogen.
4. The compound of Claim 3 wherein B is the purinyl group of Formula II.
5. The compound of Claim 3 wherein B is the pyrimidinyl group of Formula III.
6. The compound according to Claim 3 wherein R is selected from the group consisting of

| | |
|---|---|
| $CH_3C(O)$ | (acetyl) |
| $C_6H_5C(O)$ | (benzoyl) |
| $(CH_3)_3CC(O)$ | (pivaloyl) |
| $CH_3OCH_2C(O)$ | (methoxyacetyl) |
| $C_7H_{15}C(O)$ | (heptyl) |
| $C_{15}H_{31}C(O)$ | (palmityl) |
| $CH_3OC(O)$ | (methoxycarbonyl) |
| $C_8H_{17}OC(O)$ | (n-octyloxycarbonyl) |
| c-$C_6H_{11}OC(O)$ | (cyclohexyloxy carbonyl) |
| $(CH_3)_3COC(O)$ | (t-butoxycarbonyl) |
| Im-C(O) | (imidazolylcarbonyl) |

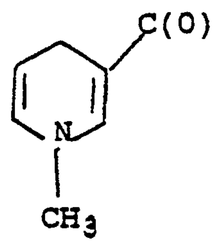

(1,4-dihydro-1-methyl-3-pyridinylcarbonyl)

7. The compound according to Claim 5 wherein B is selected from the group consisting
5-methyl-2,4-dihydroxypyrimidine (thymine),
2-hydroxy-4-aminopyrimidine (cytosine), and
2,4-dihydroxypyrimidine (uracil).

8. The compound according to Claim 7 wherein B is 5-methyl-2,4-dihydroxypyrimidine.

9. The compound according to claim 7 wherein R is selected from the group consisting of

| | |
|---|---|
| $CH_3C(O)$ | (acetyl) |
| $C_6H_5C(O)$ | (benzoyl) |
| $(CH_3)_3CC(O)$ | (pivaloyl) |
| $CH_3OCH_2C(O)$ | (methoxyacetyl) |
| $CH_3OC(O)$ | (methoxycarbonyl) |
| $C_8H_{17}OC(O)$ | (n-octyloxycarbonyl) |
| c-$C_6H_{11}OC(O)$ | (cyclohexyloxycarbonyl) |
| Im-C(O) | (imidazolylcarbonyl) |

10. A pharmaceutical composition comprising an antiviral effective amount of the compound of Claim 1 and a pharmaceutically effective carrier therefor.

11. A pharmaceutical composition according to Claim 10 wherein the compound has the formula

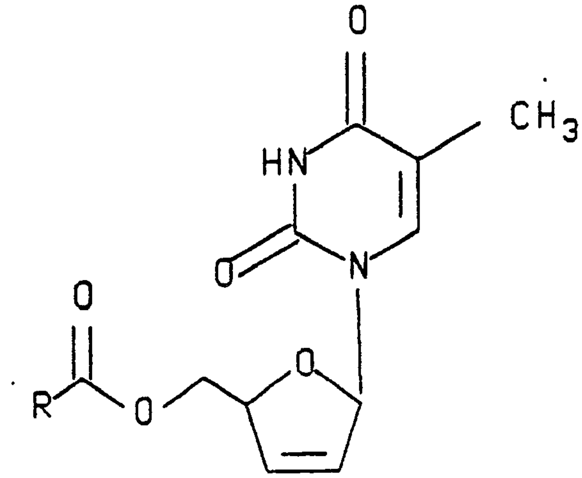

and wherein R is selected from $CH_3$-, $C_6H_5$-, $CH_3OCH_2$-, $(CH_3)_3C$-, $CH_3O$-, $C_8H_{17}O$-,

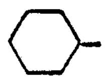 O-, 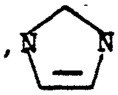 , and 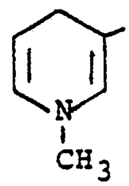 .

12. Method for preparing the compounds of claims 1 to 9 characterized in that a 2′,3′-didehydro-2′,3′-dideoxynucleoside having the general formula:

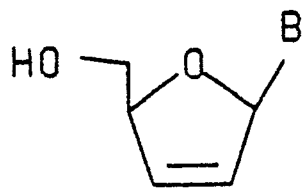

wherein B is as defined in claims 1, 4, 5, 7 and 8 is reacted with a reactive acyl compound of the general formula R -X,
wherein the acyl group R is as defined in claims 1, 6 and 9 (except for the meaning H
) and X stands for a leaving group, preferably for a halogen, in particular for C1, or for a easily cleavable acyl group forming an anhydride with R.